# EUROPÄISCHE PATENTANMELDUNG

(11) **EP 3 942 929 A1**
(43) Veröffentlichungstag der Anmeldung: **26.01.2022**
(21) Anmeldenummer: 21195283.3
(22) Anmeldetag: 29.05.2017
(51) Int. Cl.: A01M 25/00, A01M 1/20, A01M 31/00

(54) **VORRICHTUNG ZUR HALTERUNG EINES KÖDERS**

(30) Priorität: 30.05.2016 DE 102016109930
(62) Teilanmeldung aus: 17726923.0
(71) Anmelder: Buchstaller, Jürgen, 90475 Nürnberg (DE)
(72) Erfinder: Buchstaller, Jürgen, 90475 Nürnberg (DE)
(74) Vertreter: Hafner & Kohl PartmbB

(57) **Zusammenfassung**

Die Erfindung betrifft eine Vorrichtung 1 zur Halterung eines Köders 2, insbesondere eines Köders 2 für Schädlinge 14, bevorzugt für Nagetiere, umfassend:
- ein, insbesondere in einen Teil einer Kanalisationsanlage einsetzbares, Gehäuseteil 3 mit einem Aufnahmeraum 6 zur Aufnahme eines Köders 2,
- eine Erfassungseinrichtung 9, welche zur Erfassung einer ersten Information eingerichtet ist,
- eine Ausgabeeinrichtung 11, welche zur Ausgabe einer vermittels der Erfassungseinrichtung 9 erfassten ersten Information eingerichtet ist.

## Beschreibung

Die Erfindung betrifft eine Vorrichtung zur Halterung eines Köders, insbesondere eines Köders für Schädlinge, bevorzugt für Nagetiere.

Derartige Vorrichtungen dienen der Halterung von Ködern, z. B. für Schädlinge, wie Nagetiere, Insekten, etc., und sind dem Grunde nach in einer Vielzahl an unterschiedlichen Ausführungen bekannt. Die Vorrichtungen werden typischerweise zum Zwecke der Schädlingsbekämpfung, eingesetzt. Die durch die Vorrichtung gehalterten Köder enthalten entsprechend, gegebenenfalls toxische, Substanzen, welche ein Ableben der Schädlinge herbeiführen und/oder eine Vermehrung der Schädlinge verhindern.

Der Funktionsumfang entsprechender Vorrichtungen ist bis dato im Wesentlichen auf die zuverlässige Halterung von Ködern beschränkt.

Es besteht ein Bedarf, den Funktionsumfang entsprechender Vorrichtungen, insbesondere im Hinblick auf die Möglichkeit einer Erfassung spezifischer, gegebenenfalls köder- bzw. schädlingsspezifischer, Daten bzw. Informationen, deren Auswertung und Ausgabe an einen Ausgabe- bzw. Kommunikationspartner, d. h. z. B. einen Benutzer, zu erweitern.

Der Erfindung liegt die Aufgabe zugrunde, eine in ihrem Funktionsumfang erweiterte Vorrichtung zur Halterung eines Köders anzugeben.

Die Aufgabe wird durch eine Vorrichtung zur Halterung eines Köders gemäß Anspruch 1 gelöst. Die hierzu abhängigen Ansprüche betreffen mögliche Ausführungsformen der Vorrichtung. Die Aufgabe wird ferner durch ein Übertragungssystem gemäß Anspruch 18 und ein Verfahren gemäß Anspruch 19 gelöst.

Die hierin beschriebe Vorrichtung dient der Halterung wenigstens eines Köders, insbesondere wenigstens eines Köders für Schädlinge, bevorzugt für Nagetiere, Insekten, etc.. Der Köder enthält typischerweise, gegebenenfalls toxische, Substanzen, welche ein Ableben der Schädlinge herbeiführen und/oder eine Vermehrung der Schädlinge verhindern. Die Vorrichtung kann auch als Köderhaltevorrichtung bezeichnet werden.

Die Vorrichtung umfasst ein Gehäuseteil. Das Gehäuseteil ist oberirdisch und/oder unterirdisch anordenbar. Insbesondere ist das Gehäuseteil über geeignete, insbesondere gehäuseteilseitige, Befestigungseinrichtungen, insbesondere (beschädigungs- bzw. zerstörungsfrei) lösbar an oberirdischen und/oder unterirdischen Drittgegenständen befestigbar. Das Gehäuseteil ist insbesondere in eine Schacht- bzw. Kanalisationsanlage respektive in einen Teil einer solchen einsetzbar. Ein Teil einer entsprechenden Schacht- bzw. Kanalisationsanlage kann beispielhaft als ein vorgenannter Drittgegenstand erachtet werden.

Das Gehäuseteil umfasst wenigstens einen Aufnahmeraum- bzw. Innenraum. In dem Aufnahmeraum sind geeignete Halterungsmöglichkeiten zur Halterung wenigstens eines Köders angeordnet oder ausgebildet. Bei entsprechenden Halterungsmöglichkeiten kann es sich z. B. um Hängeeinrichtungen, welche eine hängende Lagerung eines Köders (in dem Aufnahmeraum) ermöglichen, oder um Plattformeinrichtungen (Köderplattform), welche eine liegende oder stehende Lagerung eines Köders (in dem Aufnahmeraum) ermöglichen, handeln. Selbstverständlich können verschiedene Halterungsmöglichkeiten zur Halterung wenigstens eines Köders, d. h. insbesondere (verschiedene) Hängeeinrichtungen und/oder (verschiedene) Plattformeinrichtungen, vorhanden sein.

Sofern eine entsprechende Plattformeinrichtung vorhanden ist, ist diese typischerweise mit wenigstens einer Durchgangsöffnung versehen, durch welche ein Schädling von einem unterhalb der Plattformeinrichtung liegenden Bereich (des Gehäuseteils) in einen oberhalb der Plattformeinrichtung liegenden Bereich (des Gehäuseteils), oder umgekehrt, gelangen kann. Der Köder befindet sich typischerweise in dem oberhalb der Plattformeinrichtung liegenden Bereich (des Gehäuseteils).

Die Vorrichtung umfasst weiterhin wenigstens eine Erfassungseinrichtung. Die Erfassungseinrichtung ist zur Erfassung einer ersten Information eingerichtet. Bei der ersten Information handelt es sich um Daten, welche über eine entsprechend hard- und/oder softwaremäßig konfigurierte Ausgabeeinrichtung ausgebbar sind oder über eine entsprechend hard- und/oder softwaremäßig konfigurierte Steuerungseinrichtung weiterverarbeitbar sind. Konkrete Beispiele für erste Informationen werden weiter unten gegeben. Zur Erfassung erster Informationen ist die Erfassungseinrichtung mit geeigneten Erfassungselementen ausgestattet. Beispiele für Erfassungselemente werden ebenso weiter unten gegeben.

Die Vorrichtung umfasst weiterhin wenigstens eine Ausgabeeinrichtung. Die Ausgabeeinrichtung ist unmittelbar oder mittelbar datenmäßig mit der Erfassungseinrichtung verbunden bzw. kommuniziert mit dieser. Die Ausgabeeinrichtung ist zur Ausgabe von vermittels der Erfassungseinrichtung erfassten ersten Informationen eingerichtet. Die vermittels der Erfassungseinrichtung erfassten ersten Informationen können über die Ausgabeeinrichtung sonach unmittelbar, d. h. ohne datenmäßige Verarbeitung, ausgegeben werden.

Die Vorrichtung kann weiterhin wenigstens eine Steuerungseinrichtung umfassen. Die Steuerungseinrichtung ist unmittelbar oder mittelbar, d. h. unter Zwischenschaltung wenigstens eines Datenübertragungsglieds, datenmäßig mit der Erfassungseinrichtung verbunden bzw. kommuniziert mit dieser. Die Steuerungseinrichtung ist auf Grundlage einer vermittels der Erfassungseinrichtung erfassten ersten Information zur Erzeugung einer zweiten Information eingerichtet. Bei der zweiten Information handelt es sich um datenverarbeitungsmäßig aus der ersten Information abgeleitete Daten, welche (im Weiteren) über eine entsprechend hard- und/oder softwaremäßig konfigurierte Ausgabeeinrichtung ausgebbar sind. Der Informationsgehalt der zweiten Information unterscheidet sich bedingt durch die datenverarbeitungsmäßige Weiterverarbeitung typischerweise von dem Informationsgehalt der ersten Information. Konkrete Beispiele für zweite Informationen werden weiter unten gegeben. Zur Erzeugung einer zweiten Information ist die Steuerungseinrichtung mit geeigneten Hard- und/oder Softwareelementen ausgestattet; mit anderen Worten ist die Steuerungseinrichtung hard- und/oder softwaremäßig zur Weiterverarbeitung der von der Erfassungseinrichtung erfassten Daten konfiguriert.

Sofern eine entsprechende Steuerungseinrichtung vorhanden ist, kann die Ausgabeeinrichtung unmittelbar oder mittelbar datenmäßig wenigstens mit der Steuerungseinrichtung verbunden sein bzw. mit dieser kommunizieren. Die Ausgabeeinrichtung ist auch zur Ausgabe von vermittels der Steuerungseinrichtung erzeugten zweiten Informationen eingerichtet.

Aus vorstehenden Ausführungen ergibt sich, dass über die Ausgabeeinrichtung erste Information, bei welchen es sich typischerweise um datenverarbeitungsmäßig nicht aufbereitete bzw. (vor)verarbeitete Rohdaten handelt, und/oder zweite Informationen, bei welchen es sich typischerweise um datenverarbeitungsmäßig aufbereitete bzw. (vor)verarbeitete Daten handelt, ausgegeben werden können. Dies bedeutet, dass vorrichtungsseitig eine datenverarbeitungsmäßige Aufbereitung bzw. (Vor)verarbeitung erster Informationen erfolgen kann, jedoch nicht muss.

Unter einer Ausgabe einer ersten bzw. zweiten Information kann eine tatsächliche bzw. unmittelbare Ausgabe der ersten bzw. zweiten Information an einer bzw. über eine Ausgabeeinrichtung verstanden werden. Ein Benutzer kann die an der Ausgabeeinrichtung ausgegebene erste bzw. zweite Information tatsächlich bzw. unmittelbar (körperlich) wahrnehmen. Eine Ausgabeeinrichtung kann entsprechend als Signalausgabeeinrichtung zur direkten Ausgabe von ersten und/oder zweiten Informationen beschreibenden Signalen an wenigstens einen Benutzer ausgebildet sein. Die Ausgabe der ersten bzw. zweiten Informationen kann z. B. über akustische und/oder haptische und/oder optische Signale erfolgen. Bei einer Signalausgabeeinrichtung kann es sich entsprechend um eine akustische Signalausgabeeinrichtung, d. h. z. B. um eine Lautsprechereinrichtung, um eine optische Signalausgabeeinrichtung, d. h. z. B. um eine Displayeinrichtung, oder um eine haptische Signalausgabeeinrichtung, d. h. z. B. um eine Vibrationseinrichtung, handeln.

Unter einer Ausgabe einer ersten bzw. zweiten Information kann jedoch auch eine Übertragung bzw. Übermittlung der ersten bzw. zweiten Information an einen mit der Ausgabeeinrichtung kommunizierenden Ausgabe- bzw. Kommunikationspartner verstanden werden. Bei einer Ausgabeeinrichtung kann es sich entsprechend um eine Übertragungs- bzw. Übermittlungseinrichtung, welche zur Übertragung bzw. Übermittlung erzeugter erster bzw. zweiter Informationen an wenigstens einen Kommunikationspartner, insbesondere an ein benutzerseitiges (mobiles) Bedien- oder Endgerät und/oder an ein lokales oder globales Datennetzwerk, z. B. ein Intranet und/oder das Internet ("Cloud"), handeln. Die Übertragung bzw. Übermittlung kann insbesondere funkbasiert erfolgen. Die Übertragungseinrichtung kann entsprechend zur funkbasierten Datenübertragung bzw. -übermittlung eingerichtet sein. Die funkbasierte Datenübertragung kann über funkbasierte Datenübertragungsmöglichkeiten bzw. -standards, d. h. z. B. Bluetooth, DECT, WLAN, etc., realisiert sein. Eine tatsächliche bzw. unmittelbare Ausgabe der ersten bzw. zweiten Information kann hier an einem Ausgabe- bzw. Kommunikationspartner, an welchen die erste bzw. zweite Information vermittels der Ausgabeeinrichtung übertragen bzw. übermittelt wurde, erfolgen. Der Kommunikationspartner kann hierfür mit geeigneten, z. B. akustischen und/oder haptischen und/oder optischen, Signalausgabeeinrichtungen ausgestattet sein. Bei dem Kommunikationspartner kann es sich, wie erwähnt, um ein benutzerseitiges (mobiles) Bedien- bzw. Endgerät handeln.

Die Erfassungseinrichtung und/oder die Steuerungseinrichtung und/oder die Ausgabeeinrichtung und/oder eine weiter unten näher erläuterte Markierungseinrichtung können einen integrierten Bestandteil des Gehäuseteils bilden. Denkbar ist es jedoch auch, dass die Erfassungseinrichtung und/oder die Steuerungseinrichtung und/oder die Ausgabeeinrichtung und/oder die weiter unten näher erläuterte Markierungseinrichtung einen integrierten Bestandteil eines mit dem Gehäuseteil verbindbaren oder verbundenen Bauteils oder eines zu dem Gehäuseteil gesonderten, d. h. räumlich-körperlich nicht mit dem Gehäuseteil verbindbaren oder verbundenen, Bauteils bilden. Die erstgenannte Variante ermöglicht eine in funktioneller Hinsicht hochintegrierte Ausbildung der Vorrichtung, die letztgenannten Varianten ermöglichen eine einfache Austauschmöglichkeit jeweiliger Einrichtungen, z. B. zum Zwecke von Service- und/oder Reparaturarbeiten, bzw. eine einfache Zugangsmöglichkeit zu den jeweiligen Einrichtungen. Bei einem mit dem Gehäuseteil verbindbaren Bauteil kann es sich um eine lösbar mit dem Gehäuseteil verbindbare Aufnahmekammer, in welcher elektrische und/oder elektronische Komponenten der Vorrichtung, insbesondere die Erfassungseinrichtung, die Steuerungseinrichtung und die Ausgabeeinrichtung, anordenbar oder angeordnet sind, handeln, welche einen Schutz dieser Komponenten gegenüber äußeren, d. h. insbesondere klimatischen und mechanischen, Einflüssen bietet.

Durch das beschriebene Prinzip der Erfassung erster Informationen, der optionalen Erzeugung zweiter Informationen auf Grundlage erfasster erster Informationen und der Ausgabe erster und/oder zweiter Informationen ist der Funktionsumfang der Vorrichtung erweitert.

Im Weiteren werden Beispiele für erste und zweite Informationen gegeben:
Eine erste Information kann eine Köderinformation sein, welche wenigstens einen Köderparameter eines Köders beschreibt bzw. beinhaltet. Über einen Köderparameter lässt sich wenigstens eine Eigenschaft eines Köders qualitativ oder quantitativ beschreiben. Selbstverständlich lässt sich über einen Köderparameter auch das Vorhandensein eines Köders qualitativ oder quantitativ beschreiben. Ein Köderparameter kann also z. B. wenigstens einen chemischen und/oder physikalischen und/oder geometrischen Parameter, d. h. z. B. die (chemische) Zusammensetzung (chemischer Parameter), das Gewicht (physikalischer Parameter), die Form oder das Volumen (geometrischer Parameter), eines Köders qualitativ oder quantitativ beschreiben. Selbstverständlich kann ein Köderparameter auch wenigstens einen aus wenigstens einem chemischen und/oder physikalischen und/oder geometrischen Parameter eines Köders abgeleiteten Parameter qualitativ oder quantitativ beschreiben bzw. beinhalten.

Alternativ oder ergänzend kann eine erste Information eine Schädlingsinformation sein, welche wenigstens einen Schädlingsparameter eines Schädlings beschreibt bzw. beinhaltet. Über einen Schädlingsparameter lässt sich wenigstens eine Eigenschaft eines Schädlings qualitativ oder quantitativ beschreiben. Selbstverständlich lässt sich über einen Schädlingsparameter auch das Vorhandensein eines Schädlings qualitativ oder quantitativ beschreiben. Ein Schädlingsparameter kann also z. B. erfasste Schädlinge und/oder Bewegungen eines oder mehrerer erfasster Schädlinge außerhalb und/oder innerhalb des gehäuseteilseitigen Aufnahmeraums qualitativ oder quantitativ beschreiben. Selbstverständlich kann ein Schädlingsparameter auch wenigstens einen aus erfassten Schädlingen und/oder Bewegungen eines oder mehrerer erfasster Schädlinge außerhalb und/oder innerhalb des gehäuseteilseitigen Aufnahmeraums abgeleiteten Parameter qualitativ oder quantitativ beschreiben bzw. beinhalten.

Eine zweite Information kann eine auf Grundlage einer erfassten Köderinformation und/oder Schädlingsinformation als Beispiele für erste Informationen erzeugte Benutzerinformation sein. Eine Benutzerinformation ist grundsätzlich eine an einen Benutzer der Vorrichtung gerichtete Information.

Die Benutzerinformation kann eine Ködergabeinformation und/oder eine Köderprüfinformation und/oder eine Schädlingsresistenzinformation beschreiben bzw. beinhalten.

Eine Ködergabeinformation kann eine, insbesondere im Hinblick auf eine bestimmte Zielgröße, insbesondere die Verhinderung der Entwicklung von Resistenzen eines Schädlings gegenüber einem bestimmten Köder, zweckmäßige, an einen Benutzer gerichtete qualitative und/oder quantitative Anweisung bzw. Empfehlung zur Einbringung eines (neuen) Köders in den gehäuseteilseitigen Aufnahmeraum beschreiben bzw. beinhalten. Einem Benutzer kann über eine entsprechende Ködergabeinformation sonach eine Anweisung bzw. Empfehlung gegeben werden, mit welchem Köder, insbesondere mit welchem in dem Köder enthaltenen Wirkstoff, er die Köderhaltevorrichtung zweckmäßigerweise (künftig) bestücken soll, um z. B. die Entwicklung von Resistenzen eines Schädlings gegenüber einem bestimmten Köder zu verhindern. Bekanntermaßen können Schädlinge bei wiederholter Gabe ein und desselben Köders - wesentlich ist hier die in dem Köder enthaltene wirksame Substanz - Resistenzen entwickeln, was die Wirksamkeit des Köders aufhebt bzw. beeinträchtigt. Dies kann durch die Gabe entsprechender Ködergabeinformationen verhindert werden.

Eine erfasste Köderinformation kann z. B. Kenntnis über die chemische Zusammensetzung eines vorrichtungsseitig gehalterten Köders oder über das, gegebenenfalls über einen bestimmten Zeitraum (un)veränderte, Gewicht bzw. Volumen des Köders geben. Die Erzeugung der Ködergabeinformation erfolgt auf Grundlage der durch die Köderinformation gegebenen Kenntnis über den Köder, d. h. z. B. dessen chemische Zusammensetzung bzw. Gewicht bzw. Volumen. Über eine entsprechende Ködergabeinformation kann sonach (gegebenenfalls) die Gabe eines unterschiedlichen Köders angewiesen werden.

In einem beispielhaften Fall kann auf Grundlage der Erfassung bzw. in Kenntnis z. B. der chemischen Zusammensetzung eines ersten Köders, eine Ködergabeinformation die Gabe eines zu dem ersten Köder im Hinblick auf seine chemische Zusammensetzung verschiedenen zweiten Köders anweisen. In einem weiteren beispielhaften Fall kann auf Grundlage der Erfassung bzw. in Kenntnis eines über einen bestimmten Zeitraum wenig oder nicht veränderten Gewichts bzw. Volumens eines Köders, wodurch angezeigt wird, dass der Köder von den Schädlingen nicht angenommen wird, eine Ködergabeinformation die Gabe eines zu dem ersten Köder im Hinblick auf seine chemische Zusammensetzung verschiedenen zweiten Köders anweisen, welcher von den Schädlingen gegebenenfalls bessre angenommen wird.

Eine Köderprüfinformation kann eine, insbesondere an eine bestimmte Zeitdauer oder einen bestimmten Zeitpunkt geknüpfte, an einen Benutzer gerichtete qualitative und/oder quantitative Anweisung zur Überprüfung eines gegebenen Köders, insbesondere im Hinblick auf durch einen Köderanbiss durch einen Schädling zurückzuführende Veränderungen des Köders, beschreiben bzw. beinhalten. Einem Benutzer kann über eine entsprechende Köderprüfinformation sonach eine Anweisung bzw. eine Empfehlung gegeben werden, wann bzw. in welchen regelmäßigen oder unregelmäßigen Zeiträumen, er eine Überprüfung eines gegebenen Köders, insbesondere im Hinblick auf durch einen Köderanbiss durch einen Schädling zurückzuführende Veränderungen des Köders, vornehmen soll. Typischerweise wirken Köder innerhalb eines bestimmten Zeitraums, nach Ablauf dessen eine (deutliche) Abnahme des Schädlingsbefalls zu erwarten ist. Dem Benutzer kann nach Gabe eines bestimmten Köders sonach in Abhängigkeit des köderspezifischen Zeitraums, nach Ablauf dessen eine (deutliche) Abnahme des Schädlingsbefalls zu erwarten ist, über eine Köderprüfinformation angewiesen werden, eine Überprüfung des vorrichtungsseitig gehalterten Köders vorzunehmen. Eine Köderprüfinformation kann grundsätzlich. ein bestimmtes Zeitintervall (Prüfrhythmus) bzw einen bestimmten Zeitraum bzw. einen bestmimten Zeitpunkt (Uhrzeit, Tag, Monat, Jahr) enthalten, innerhalb dessen bzw. zu welchem er eine Überprüfung des Köders vornehmen soll. Dem Benutzer können auch Prüfintervalle gegeben werden, zu welchen er eine Überprüfung des Köders vornehmen soll.

Eine Schädlingsresistenzinformation kann ein aktuelles und/oder künftiges Potential der Entwicklung von Resistenzen eines Schädlings gegenüber einem bestimmten, gegebenenfalls bereits in der Vergangenheit ein- oder mehrfach gegebenen, Köder beschreiben bzw. beinhalten. Einem Benutzer kann über eine entsprechende Schädlingsresistenzinformation sonach eine Information über aktuell und/oder künftig gegebene Resistenzen gegeben werden, sodass dieser die Auswahl eines Köders zur Bestückung der Köderhaltevorrichtung entsprechend variiert, um aktuell bzw. künftig gegebenen Resistenzen entgegen zu wirken. In einem beispielhaften Fall kann auf Grundlage der Erfassung bzw. die Kenntnis einer über einen bestimmten Zeitraum unveränderten (oder gestiegenen) Anzahl an Schädlingen in dem Gehäuseteil eine Schädlingsresistenzinformation auf eine (entwickelte) Resistenz der Schädlinge gegenüber einem bestimmten, nämlich dem in dem Aufnahmeraum aufgenommenen Köder, hinweisen.

Es wurde erwähnt, dass die Erfassungseinrichtung zur Erfassung erster Informationen, d. h. insbesondere zur Erfassung von Köderinformationen bzw. Schädlingsinformationen, mit geeigneten Erfassungselementen ausgestattet ist. Die Erfassungseinrichtung umfasst sonach wenigstens ein Erfassungselement zur Erfassung einer ersten Information, d. h. insbesondere zur Erfassung einer Köderinformation bzw. einer Schädlingsinformation.

Ein zur Erfassung einer Köderinformation eingerichtetes Erfassungselement kann eine Sensoreinrichtung zur Erfassung eines chemischen und/oder physikalischen und/oder geometrischen Köderparameters sein. Die Sensoreinrichtung kann z. B. zur Erfassung bestimmter, gegebenenfalls (leicht) flüchtiger, Substanzen eines Köders eingerichtet sein, sodass eine Erfassung eines chemischen Köderparameters möglich ist. Weiterhin kann die oder eine (weitere) Sensoreinrichtung z. B. zur Erfassung des Gewichts eines Köders eingerichtet sein, sodass eine Erfassung eines physikalischen Köderparameters möglich ist. Überdies kann die oder eine (weitere) Sensoreinrichtung z. B. zur Erfassung der Form bzw. des Volumens eines Köders eingerichtet sein, sodass eine Erfassung eines geometrischen Köderparameters möglich ist.

Ein zur Erfassung einer Köderinformation eingerichtetes Erfassungselement kann auch eine Leseeinrichtung zur Auslesung einer einen Köderparameter enthaltenden, insbesondere köderseitigen, Speichereinrichtung (Speicherchip, Tag, etc.) sein. Die Leseeinrichtung ist datenmäßig mit dem Köder bzw. einer einen Köderparameter enthaltenden Speichereinrichtung verbindbar bzw. verbunden, sodass ein Auslesen des Köderparameters möglich ist. Ein Auslesen ist z. B. in dem ordnungsgemäß durch die Vorrichtung gehalterten Zustand des Köders möglich. Es ist sonach nicht zwingend notwendig, einen Köderparameter vorrichtungsseitig sensorisch zu erfassen, vielmehr kann ein Köderparameter von der Erfassungseinrichtung ausgelesen und derart erfasst werden.

Ein zur Erfassung einer Köderinformation eingerichtetes Erfassungselement kann auch eine Empfangseinrichtung zum Empfang eines, insbesondere von einem Benutzer (vor, während oder nach der Einbringung eines Köders in den gehäuseteilseitigen Aufnahmeraum), übertragenen Köderparameters sein. Die Empfangseinrichtung, welche einen Teil einer vorrichtungsseitigen Ausgabeeinrichtung in Form einer Übertragungs- bzw. Übermittlungseinrichtung bilden kann, ist sonach datenmäßig mit einer, insbesondere benutzerseitigen Sendeeinrichtung, welche einen Teil eines benutzerseitigen (mobilen) Bedien- bzw. Endgeräts bilden kann, verbindbar bzw. bzw. verbunden, sodass ein Empfang eines Köderparameters möglich ist. Es ist sonach auch hier nicht zwingend notwendig, einen Köderparameter vorrichtungsseitig sensorisch zu erfassen, vielmehr kann ein Köderparameter an die Erfassungseinrichtung übertragen werden bzw. von dieser empfangen und derart erfasst werden.

Ein zur Erfassung einer Schädlingsinformation eingerichtetes Erfassungselement kann eine Sensoreinrichtung zur Erfassung von Schädlingen und/oder zur Erfassung von Bewegungen von Schädlingen außerhalb und/oder innerhalb des gehäuseteilseitigen Aufnahmeraums sein. Die Sensoreinrichtung kann z. B. zur Erfassung einer Atmosphäre und/oder einer Temperatur außerhalb und/oder innerhalb des Aufnahmeraums eingerichtet sein, sodass eine Erfassung eines Schädlingsparameters möglich ist. Beispielsweise kann eine Erfassung einer Atmosphäre mit einer gegenüber einer Referenzatmosphäre veränderten Zusammensetzung (beispielsweise einem erhöhten CO₂-Gehalt) bzw. eine Erfassung einer gegenüber einer Referenztemperatur veränderten Temperatur das Vorhandensein von Schädlingen anzeigen. Die Sensoreinrichtung umfasst hierfür geeignete Gas- bzw. Temperatursensoren. Die Sensoreinrichtung kann jedoch auch zur Erfassung von auf eine Fläche, d. h. z. B. eine Fläche einer Wandung des Gehäuseteils bzw. eine Fläche einer gehäuseteilseitigen Plattformeinrichtung, wirkenden Kräften, insbesondere Druck- bzw. Zugkräften, eingerichtet sein, sodass derart eine Erfassung eines Schädlingsparameters möglich ist. Beispielsweise kann eine Erfassung eines auf eine bestimmte Fläche wirkenden Drucks oder Zugs das Vorhandensein von Schädlingen anzeigen. Die Sensoreinrichtung umfasst hierfür geeignete Druck- bzw. Zugsensoren.

Ein zur Erfassung einer Schädlingsinformation eingerichtetes Erfassungselement kann auch eine Sensoreinrichtung zur Erfassung einer schädlingsseitigen, d. h. äußerlich oder innerlich mit einem Schädling verbundenen, insbesondere biologischen, chemischen, magnetischen oder mechanischen, Markereinrichtung (Marker) sein. Die Sensoreinrichtung kann sonach bestimmte schädlingsseitige Markereinrichtungen, welche Kenntnisse über das Vorhandensein bzw. Bewegungsmuster der mit diesen verbundenen Schädlinge liefern, erfassen. Biologische bzw. chemische Markereinrichtungen können bestimmte biologische oder chemische Substanzen sein, welche einem Schädling äußerlich oder innerlich zugeführt worden sind. Beispielsweise kann die Oberfläche eines Schädlings mit einer entsprechenden Substanz benetzt sein. Magnetische oder mechanische Markereinrichtungen können z. B. magnetisch oder mechanisch, d. h. durch einen mechanischen Kontakt, erfassbare magnetische oder mechanische Elemente sein, welche einem Schädling äußerlich oder innerlich zugeführt worden sind. Beispielsweise kann ein entsprechendes Element an einem Körperteil eines Schädlings befestigt sein.

Zur Markierung eins Schädlings mit einer entsprechend Markereinrichtung kann die Vorrichtung eine Markierungseinrichtung umfassen. Die Markierungseinrichtung ist zur Markierung eines Schädlings mit einer, insbesondere biologischen, chemischen, magnetischen oder mechanischen, von der Sensoreinrichtung zur Erfassung einer schädlingsseitigen Markereinrichtung erfassbaren Markereinrichtung eingerichtet. Die Markierung kann - je nach den strukturellen Gegebenheiten der jeweiligen Markereinrichtung - z. B. durch Aufsprühen, Berühren eines mit einer Markereinrichtung versehenen, z. B. bürstenartigen, Kontaktbereichs, Spritzen, Anbinden, Ankleben, Anheften, etc. erfolgen.

Ein zur Erfassung einer Schädlingsinformation eingerichtetes Erfassungselement kann auch eine Leseeinrichtung zur Auslesung einer einen Schädlingsparameter enthaltenden, insbesondere schädlingsseitigen, Speichereinrichtung (Speicherchip, Tag, etc.) sein. Die Leseeinrichtung ist datenmäßig mit dem Schädling bzw. einer einen Schädlingsparameter enthaltenden Speichereinrichtung verbindbar bzw. verbunden, sodass ein Auslesen des Schädlingsparameters möglich ist. Ein Auslesen ist z. B. möglich, wenn sich ein Schädlings innerhalb eines Lesebereichs befindet. Ein solcher Lesebereich kann sich außerhalb oder innerhalb des Gehäuseteils befinden.

Ein zur Erfassung einer Schädlingsinformation eingerichtetes Erfassungselement kann auch eine Aufnahmeeinrichtung, insbesondere eine Bild- und/oder Tonaufnahmeeinrichtung, zur Aufnahme von Schädlingen und/oder zur Aufnahme von Bewegungen von Schädlingen außerhalb und/oder innerhalb des gehäuseteilseitigen Aufnahmeraums sein. Die Aufnahmeeinrichtung kann z. B. zur Aufnahme von akustischen und/oder optischen Informationen außerhalb und/oder innerhalb des Aufnahmeraums eingerichtet sein, sodass eine Erfassung eines Schädlingsparameters möglich ist. Beispielsweise kann eine Aufnahme eines bestimmten Geräuschpegels oder Bewegungsmusters das Vorhandensein von Schädlingen anzeigen. Die Aufnahmeeinrichtung umfasst hierfür geeignete akustische und/oder optische Aufnahmeelemente, d. h. z. B. Mikrophone und/oder Kameras.

Die im Zusammenhang mit der Erfassung von Köder- wie auch Schädlingsinformationen genannten Erfassungselemente, insbesondere die genannten Sensoreinrichtungen und Aufnahmeeinrichtungen, können eine dynamische, d. h. örtlich und/oder zeitlich aufgelöste, Erfassung von Köder bzw. Schädlingsparametern ermöglichen.

Die Vorrichtung kann eine Speichereinrichtung (Datenspeicher) umfassen. Die Speichereinrichtung ist zur Speicherung vermittels der Erfassungseinrichtung erfasster erster Informationen, d. h. insbesondere Köder- bzw. Schädlingsinformationen, und/oder zur Speicherung vermittels der Steuerungseinrichtung erzeugter zweiter Informationen, d. h. insbesondere Benutzerinformationen, insbesondere Ködergabe- und Schädlingsresistenzinformationen, eingerichtet. Die Speicherung entsprechender erster und zweiter Informationen ist im Hinblick auf die beschriebene Funktionserweiterung der Vorrichtung zweckmäßig.

Die Vorrichtung kann eine Schließeinrichtung umfassen, welche einen, insbesondere an dem Gehäuseteil, relativ zu dem Gehäuseteil zwischen einer Offenstellung und einer Schließstellung bewegbar gelagerten Schließkörper umfasst. Der Schließkörper ist in der Offenstellung derart von einem Zugangsbereich des Gehäuseteils in den gehäuseteilseitigen Aufnahmeraum weg bewegt, dass der Zugangsbereich geöffnet ist. In der Schließstellung ist der Schließkörper derart gegen den Zugangsbereich bewegt, dass der Zugangsbereich, insbesondere hermetisch, verschlossen ist. in der Schließstellung ist eine Kontamination der Umwelt mit den in dem Köder enthaltenen Substanzen ausgeschlossen. Bei dem Schließkörper kann es sich z. B. um einen Schwimmkörper handeln bzw. kann der Schließkörper einen solchen umfassen.

Die Erfindung betrifft ferner ein Übertragungssystem zur Übertragung von ersten Informationen, insbesondere Köder- bzw. Schädlingsinformationen, und/oder zweiten Informationen, insbesondere Benutzerinformationen, bevorzugt Ködergabeinformationen und/oder Schädlingsresistenzinformationen, an wenigstens einen Übertragungs- bzw. Kommunikationspartner. Das Übertragungssystem umfasst wenigstens eine wie beschriebene Vorrichtung sowie wenigstens einen Übertragungspartner, welcher wenigstens zum Empfang von von der vorrichtungsseitigen Ausgabeeinrichtung übertragener zweiter Informationen eingerichtet ist. Bei dem Übertragungspartner kann es sich insbesondere um ein benutzerseitiges (mobiles) End- bzw. Bediengerät, d. h. insbesondere um ein Laptop, ein Smartphone, ein Tablet-PC oder ein sonstiges mobiles elektronisches Gerät handeln.

Die Erfindung betrifft ferner ein Verfahren zur Übertragung von ersten Informationen, insbesondere Köder- bzw. Schädlingsinformationen, und/oder zweiten Informationen, insbesondere Benutzerinformationen, bevorzugt Ködergabeinformationen und/oder Schädlingsresistenzinformationen, an wenigstens einen Übertragungs- bzw. Kommunikationspartner.

Das Verfahren umfasst in einer ersten Ausgestaltung die folgenden Schritte:
- Erfassen einer ersten Information, insbesondere einer wenigstens einen Köderparameter eines Köders beschreibenden Köderinformation und/oder einer wenigstens einen Schädlingsparameter eines Schädlings beschreibenden Schädlingsinformation, vermittels der Erfassungseinrichtung einer wie beschriebenen Vorrichtung und
- Übertragen der erfassten ersten Information an wenigstens einen Übertragungspartner vermittels der Ausgabeeinrichtung der Vorrichtung.

Die datenmäßige Verarbeitung der ersten Informationen kann hier außerhalb der Vorrichtung erfolgen.

Das Verfahren umfasst einer zweiten Ausgestaltung die folgenden Schritte:
- Erfassen einer ersten Information, insbesondere einer wenigstens einen Köderparameter eines Köders beschreibenden Köderinformation und/oder einer wenigstens einen Schädlingsparameter eines Schädlings beschreibenden Schädlingsinformation, vermittels der Erfassungseinrichtung einer wie beschriebenen Vorrichtung,
- Erzeugen einer zweiten Information, insbesondere einer Benutzerinformation, auf Grundlage einer erfassten ersten Information, insbesondere einer Köderinformation und/oder einer erfassten Schädlingsinformation, vermittels der Steuerungseinrichtung der Vorrichtung und
- Übertragen der erzeugten zweiten Information an wenigstens einen Übertragungspartner vermittels der Ausgabeeinrichtung der Vorrichtung.

Die datenmäßige Verarbeitung der ersten Informationen kann hier (auch) innerhalb der Vorrichtung erfolgen.

Bei dem Übertragungs- bzw. Kommunikationspartner kann es sich, wie erwähnt, insbesondere um ein benutzerseitiges (mobiles) End- bzw. Bediengerät, d. h. insbesondere um ein Laptop, ein Smartphone, ein Tablet-PC oder ein sonstiges mobiles elektronisches Gerät handeln.

Sämtliche Ausführungen im Zusammenhang mit der Vorrichtung gelten analog sowohl für das Übertragungssystem als auch für das Verfahren und umgekehrt.

Die Erfindung ist anhand von Ausführungsbeispielen in den Zeichnungsfiguren näher erläutert. Dabei zeigen:

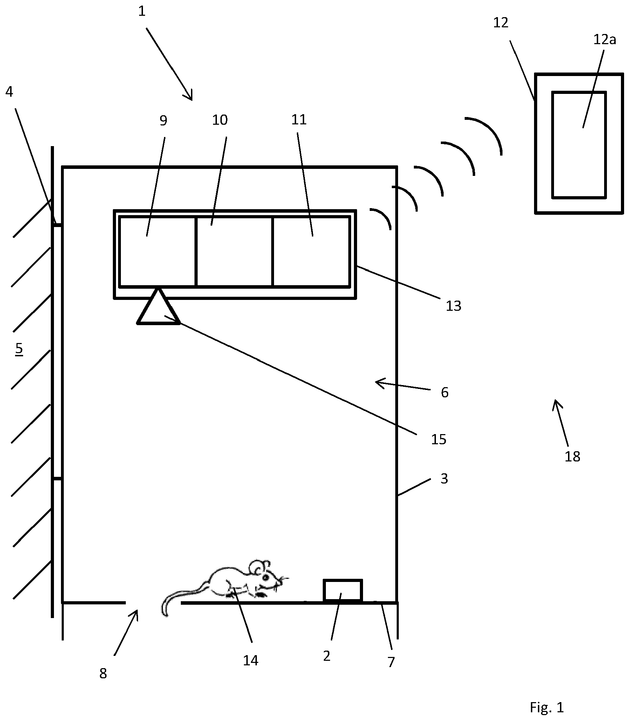

Fig. 1 - 4 je eine Prinzipdarstellung einer Vorrichtung zur Halterung eines Köders gemäß einem Ausführungsbeispiel;

Die Fig. zeigen jeweils eine Prinzipdarstellung einer Vorrichtung 1 zur Halterung eines Köders 2 gemäß einem Ausführungsbeispiel. Einzelne, mehrere oder sämtliche Merkmale der in den einzelnen Ausführungsbeispielen gezeigten Vorrichtungen 1 können kombiniert werden.

Die Vorrichtung 1 dient der Halterung eines Köders 2, insbesondere wenigstens eines Köders 2 für Schädlinge 14, bevorzugt für Nagetiere, wie Ratten oder Mäuse, oder Insekten, wie Maden, Schaben, etc. Der Köder 2 enthält, gegebenenfalls toxische, Substanzen, welche ein Ableben der Schädlinge 14 herbeiführen und/oder eine Vermehrung der Schädlinge 14 verhindern.

Die Vorrichtung 1 umfasst ein Gehäuseteil 3. Das Gehäuseteil 3 ist oberirdisch und/oder unterirdisch anordenbar. Das Gehäuseteil 3 ist mit rein schematisch angedeuteten Befestigungseinrichtungen 4 ausgestattet, über welche es (lösbar) an oberirdischen und/oder unterirdischen Drittgegenständen 5 befestigbar ist. Das Gehäuseteil 3 kann derart z. B. in eine, insbesondere unterirdische, Schacht- bzw. Kanalisationsanlage (nicht gezeigt) respektive in einen Teil einer solchen einsetzbar sein. Ein Teil einer entsprechenden Schacht- bzw. Kanalisationsanlage kann entsprechend als Drittgegenstand 5 erachtet werden.

Ersichtlich umfasst das Gehäuseteil 3 einen Aufnahme- bzw. Innenraum 6. In dem Aufnahmeraum 6 ist eine geeignete Halterungsmöglichkeit zur Halterung des Köders 2 angeordnet oder ausgebildet. Bei der Halterungsmöglichkeit handelt es sich bei den in den Fig. gezeigten Ausführungsbeispielen um eine Plattformeinrichtung 7 (Köderplattform), welche eine liegende oder stehende Lagerung des Köders 2 ermöglicht. Die Plattformeinrichtung 7 ist mit einer Durchgangsöffnung 8 versehen, durch welche ein Schädling 14 von einem unterhalb der Plattformeinrichtung 7 liegenden Bereich des Gehäuseteils 3 in einen oberhalb der Plattformeinrichtung 7 liegenden Bereich des Gehäuseteils 3, oder umgekehrt, gelangen kann. Ersichtlich befindet sich der Köder 2 in dem oberhalb der Plattformeinrichtung 7 liegenden Bereich des Gehäuseteils 3.

Wenngleich in den Fig. nicht gezeigt, könnte es sich bei einer alternativen oder ergänzenden Halterungsmöglichkeit auch um eine Hängeeinrichtung (nicht gezeigt), welche eine hängende Lagerung des Köders 2 ermöglicht, handeln.

Die Vorrichtung 1 umfasst weiterhin eine Erfassungseinrichtung 9. Die Erfassungseinrichtung 9 ist zur Erfassung erster Informationen eingerichtet. Bei ersten Informationen handelt es sich im Allgemeinen um Daten, welche über eine entsprechend hard- und/oder softwaremäßig konfigurierte Steuerungseinrichtung 10 weiterverarbeitbar sind.

In dem in den Fig. 1, 3 und 4 gezeigten Ausführungsbeispielen umfasst ie Vorrichtung 1 weiterhin eine Steuerungseinrichtung 10. Die Steuerungseinrichtung 10 ist datenmäßig mit der Erfassungseinrichtung 9 verbunden und kommuniziert mit dieser. Die Steuerungseinrichtung 10 ist auf Grundlage einer vermittels der Erfassungseinrichtung 9 erfassten ersten Information zur Erzeugung einer zweiten Information eingerichtet. Bei einer zweiten Information handelt es sich im Allgemeinen um datenverarbeitungsmäßig aus einer ersten Information abgeleitete Daten, welche (im Weiteren) über eine entsprechend hard- und/oder softwaremäßig konfigurierte Ausgabeeinrichtung 11 ausgebbar sind. Der Informationsgehalt der zweiten Information unterscheidet sich bedingt durch die datenverarbeitungsmäßige Weiterverarbeitung von dem Informationsgehalt der ersten Information. Zur Erzeugung zweiter Informationen ist die Steuerungseinrichtung 10 mit geeigneten Hard- und/oder Softwareelementen (nicht gezeigt) ausgestattet; mithin ist die Steuerungseinrichtung 10 hard- und/oder softwaremäßig zur Weiterverarbeitung der von der Erfassungseinrichtung 9 erfassten Daten konfiguriert.

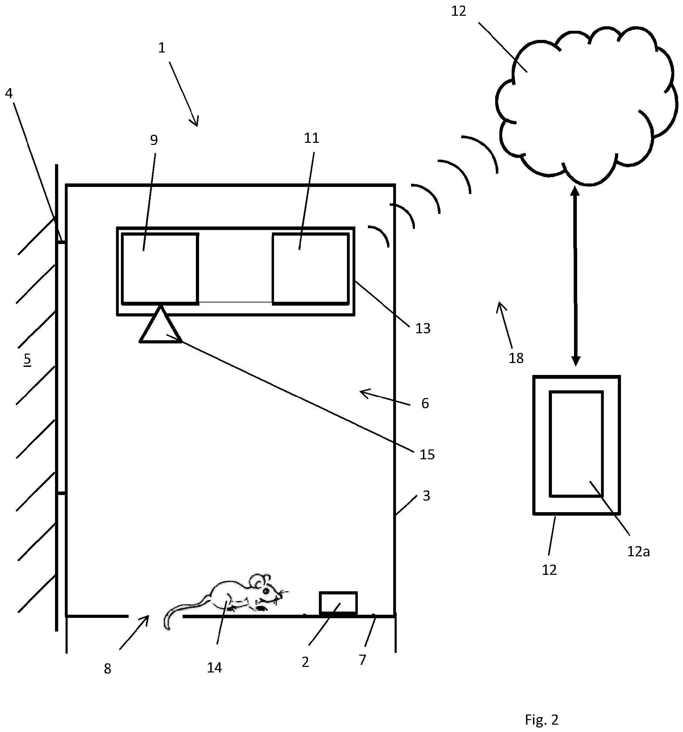

In dem in Fig. 2 gezeigten Ausführungsbeispiel umfasst die Vorrichtung 1 keine Steuerungseinrichtung 10, eine datenmäßige Verarbeitung der vermittels der Erfassungseinrichtung 9 erfassten ersten Informationen ist hier vorrichtungsseitig nicht vorgesehen. Wie sich im Weiteren ergibt, erfolgt die datenmäßige Verarbeitung der vermittels der Erfassungseinrichtung 9 erfassten ersten Informationen hier seitens eines Kommunikationspartners 12.

Die Vorrichtung 1 umfasst in allen Ausführungsbeispielen eine Ausgabeeinrichtung 11. Die Ausgabeeinrichtung 11 ist mit der Steuerungseinrichtung 10 - sofern vorhanden - verbunden bzw. kommuniziert mit dieser. Die Ausgabeeinrichtung 11 ist zur Ausgabe von vermittels der Erfassungseinrichtung 9 erfassten ersten Informationen bzw. von vermittels der Steuerungseinrichtung 10 erzeugten zweiten Informationen eingerichtet. In den in Fig. gezeigten Ausführungsbeispielen ist unter einer Ausgabe einer ersten bzw. zweiten Information eine Übertragung bzw. Übermittlung der ersten bzw. zweiten Information an einen mit der Ausgabeeinrichtung 11 kommunizierenden Kommunikationspartner 12 zu verstehen. Bei der Ausgabeeinrichtung 11 handelt es sich entsprechend um eine Übertragungs- bzw. Übermittlungseinrichtung, welche zur Übertragung bzw. Übermittlung erzeugter erster bzw. zweiter Informationen an wenigstens einen Kommunikationspartner 12 eingerichtet ist. Bei dem Kommunikationspartner 12 handelt es sich in den Ausführungsbeispielen gemäß den Fig. 1, 3, 4 um ein benutzerseitiges (mobiles) Bedien- oder Endgerät und bei dem Ausführungsbeispiel gemäß Fig. 2 um ein lokales oder globales Datennetzwerk, d. h. z. B. ein Intranet und/oder das Internet ("Cloud"). Selbstverständlich kann ein benutzerseitiges (mobiles) Bedien- oder Endgerät datenmäßig mit dem Datennetzwerk verbunden sein.

Eine Verarbeitung der an das einen Kommunikationspartner 12 darstellenden Datennetzwerk übermittelten ersten Informationen kann über mit diesem kommunizierende Datenverarbeitungseinrichtungen erfolgen ("Cloud-Computing"). Selbstverständlich kann seitens des Datennetzwerks auch eine Weiterverarbeitung von an dieses übermittelten zweiten Informationen erfolgen.

Die Ausgabeeinrichtung 11 ist zur funkbasierten Datenübertragung bzw. -übermittlung eingerichtet. Die funkbasierte Datenübertragung ist über funkbasierte Datenübertragungsmöglichkeiten bzw. -standards, d. h. z. B. Bluetooth, DECT, WLAN, etc., realisiert. Der DECT-Standard hat sich als besonders zuverlässig für eine funkbasierte Datenübertragung aus unterirdischen Schacht- oder Kanalisationsanlagen erwiesen und ist insofern für unterirdisch angeordnete Vorrichtungen 1 zweckmäßig.

Eine tatsächliche bzw. unmittelbare Ausgabe zweiter Informationen kann an dem Kommunikationspartner 12, an welchen die zweiten Informationen vermittels der Ausgabeeinrichtung 11 übertragen wurde, erfolgen. Der Kommunikationspartner 12 kann hierfür mit geeigneten, z. B. akustischen und/oder haptischen und/oder optischen, Signalausgabeeinrichtungen 12a, d. h. z. B. Display- und/oder Lautsprechereinrichtungen, ausgestattet sein.

Wenngleich in den Fig. nicht gezeigt, kann die Ausgabeeinrichtung 11 auch als Signalausgabeeinrichtung zur direkten Ausgabe von ersten und/oder zweiten Informationen beschreibenden Signalen an einen Benutzer ausgebildet sein. Bei einer solchen Signalausgabeeinrichtung kann es sich um eine akustische Signalausgabeeinrichtung, d. h. z. B. um eine Lautsprechereinrichtung, um eine optische Signalausgabeeinrichtung, d. h. z. B. um eine Displayeinrichtung, oder um eine haptische Signalausgabeeinrichtung, d. h. z. B. um eine Vibrationseinrichtung, handeln. Unter einer Ausgabe einer zweiten Information kann sonach prinzipiell auch eine tatsächliche bzw. unmittelbare Ausgabe der zweiten Information an der bzw. über die Ausgabeeinrichtung 11 verstanden werden.

Die Erfassungseinrichtung 9, die Steuerungseinrichtung 10 und die Ausgabeeinrichtung 11 können jeweils einen integrierten Bestandteil des Gehäuseteils 3 bilden. Denkbar ist es jedoch auch, dass die Erfassungseinrichtung 9, die Steuerungseinrichtung 10 und die Ausgabeeinrichtung 11 einen integrierten Bestandteil eines mit dem Gehäuseteil 3 verbindbaren oder verbundenen Bauteils 13 bilden. Bei dem Bauteil 13 kann es sich um eine lösbar mit dem Gehäuseteil 3 verbindbare Aufnahmekammer, in welcher elektrische und/oder elektronische Komponenten der Vorrichtung 1, d. h. insbesondere die Erfassungseinrichtung 9, die Steuerungseinrichtung 10 und die Ausgabeeinrichtung 11, angeordnet sind, handeln.

Eine erste Information kann eine Köderinformation sein, welche wenigstens einen Köderparameter des Köders 2 beschreibt bzw. beinhaltet. Ein Köderparameter kann z. B. einen chemischen und/oder physikalischen und/oder geometrischen Parameter, d. h. z. B. die (chemische) Zusammensetzung (chemischer Parameter), das Gewicht (physikalischer Parameter), die Form oder das Volumen (geometrischer Parameter), des Köders 2 qualitativ oder quantitativ beschreiben.

Alternativ oder ergänzend kann eine erste Information eine Schädlingsinformation sein, welche wenigstens einen Schädlingsparameter eines Schädlings 14 beschreibt bzw. beinhaltet. Ein Schädlingsparameter kann z. B. erfasste Schädlinge 14 und/oder Bewegungen eines oder mehrerer erfasster Schädlinge 14 außerhalb und/oder innerhalb des Aufnahmeraums 6 qualitativ oder quantitativ beschreiben.

Eine zweite Information kann eine auf Grundlage einer erfassten Köderinformation und/oder Schädlingsinformation erzeugte Benutzerinformation sein. Die Benutzerinformation kann eine Ködergabeinformation und/oder Köderprüfinformation und/oder eine Schädlingsresistenzinformation beschreiben bzw. beinhalten.

Die Ködergabeinformation kann eine, insbesondere im Hinblick auf eine bestimmte Zielgröße, insbesondere die Verhinderung der Entwicklung von Resistenzen eines Schädlings 14 gegenüber einem bestimmten Köder 2, zweckmäßige, an einen Benutzer gerichtete qualitative und/oder quantitative Anweisung bzw. Empfehlung zur Einbringung eines (neuen) Köders 2 in den Aufnahmeraum 6 beschreiben bzw. beinhalten. Einem Benutzer kann über eine entsprechende Ködergabeinformation sonach eine Anweisung bzw. Empfehlung gegeben werden, mit welchem Köder 2, insbesondere mit welchem in dem Köder 2 enthaltenen Wirkstoff, er die Vorrichtung 1 zweckmäßigerweise (künftig) bestücken soll, um z. B. die Entwicklung von Resistenzen eines Schädlings 14 gegenüber einem bestimmten Köder 2 zu verhindern. Die Entwicklung von Resistenzen kann durch die Gabe entsprechender Ködergabeinformationen verhindert werden.

Eine erfasste Köderinformation kann z. B. Kenntnis über die chemische Zusammensetzung eines vorrichtungsseitig gehalterten Köders 2 oder über das, gegebenenfalls über einen bestimmten Zeitraum (un)veränderte, Gewicht bzw. Volumen des Köders 2 geben. Die Erzeugung der Ködergabeinformation erfolgt auf Grundlage der durch die Köderinformation gegebenen Kenntnis über den Köder 2, d. h. z. B. dessen chemische Zusammensetzung bzw. Gewicht bzw. Volumen. Über eine entsprechende Ködergabeinformation kann sonach (gegebenenfalls) die Gabe eines unterschiedlichen Köders 2 angewiesen werden.

Beispielsweise kann auf Grundlage der Erfassung bzw. in Kenntnis z. B. der chemischen Zusammensetzung eines ersten Köders 2, eine Ködergabeinformation die Gabe eines zu dem ersten Köder 2 im Hinblick auf seine chemische Zusammensetzung verschiedenen zweiten Köders 2 anweisen.

Weiter beispielsweise kann auf Grundlage der Erfassung bzw. in Kenntnis eines über einen bestimmten Zeitraum wenig oder nicht veränderten Gewichts bzw. Volumens des Köders 2, wodurch angezeigt wird, dass der Köder 2 von den Schädlingen 14 nicht angenommen wird, eine Ködergabeinformation die Gabe eines zu dem gegebenen Köder 2 im Hinblick auf seine chemische Zusammensetzung verschiedenen zweiten Köders 2 anweisen, welcher von den Schädlingen 14 gegebenenfalls besser angenommen wird.

Die Köderprüfinformation ist eine, insbesondere an eine bestimmte Zeitdauer oder einen bestimmten Zeitpunkt geknüpfte, an einen Benutzer gerichtete qualitative und/oder quantitative Anweisung zur Überprüfung eines gegebenen Köders 2, insbesondere im Hinblick auf durch einen Köderanbiss durch einen Schädling 14 zurückzuführende Veränderungen des Köders 2, beschreiben bzw. beinhalten. Einem Benutzer kann über eine entsprechende Köderprüfinformation sonach eine Anweisung bzw. eine Empfehlung gegeben werden, wann bzw. in welchen regelmäßigen oder unregelmäßigen Zeiträumen, er eine Überprüfung eines gegebenen Köders 2, insbesondere im Hinblick auf durch einen Köderanbiss durch einen Schädling 14 zurückzuführende Veränderungen des Köders 2, vornehmen soll. Eine Köderprüfinformation kann grundsätzlich. ein bestimmtes Zeitintervall (Prüfrhythmus) bzw. einen bestimmten Zeitraum bzw. einen bestmimten Zeitpunkt (Uhrzeit, Tag, Monat, Jahr) enthalten, innerhalb dessen bzw. zu welchem er eine Überprüfung des Köders 2 vornehmen soll. Dem Benutzer können auch Prüfintervalle gegeben werden, zu welchen er eine Überprüfung des Köders 2 vornehmen soll.

Die Schädlingsresistenzinformation kann ein aktuelles und/oder künftiges Potential der Entwicklung von Resistenzen eines Schädlings 14 gegenüber einem bestimmten, gegebenenfalls bereits in der Vergangenheit ein- oder mehrfach gegebenen, Köder 2 beschreiben bzw. beinhalten. Einem Benutzer kann über eine entsprechende Schädlingsresistenzinformation sonach eine Information über aktuell und/oder künftig (möglicherweise) gegebene Resistenzen gegeben werden, sodass dieser die Auswahl eines Köders 2 zur Bestückung der Vorrichtung 1 entsprechend variiert, um aktuell bzw. künftig gegebenen Resistenzen entgegen zu wirken. Beispielsweise Fall kann über die Erfassung bzw. die Kenntnis einer über einen bestimmten Zeitraum unveränderten (oder gestiegenen) Anzahl an Schädlingen 14 in dem Gehäuseteil 3 eine Schädlingsresistenzinformation auf eine (entwickelte) Resistenz der Schädlinge 14 gegenüber einem bestimmten, nämlich dem in dem Aufnahmeraum 6 aufgenommenen Köder 2, hinweisen.

Ein zur Erfassung einer Köderinformation eingerichtetes Erfassungselement (nicht gezeigt) als Teil der Erfassungseinrichtung 9 kann eine Sensoreinrichtung zur Erfassung eines chemischen und/oder physikalischen und/oder geometrischen Köderparameters sein. Die Sensoreinrichtung kann z. B. zur Erfassung bestimmter, gegebenenfalls (leicht) flüchtiger, Substanzen des Köders 2 eingerichtet sein, sodass eine Erfassung eines chemischen Köderparameters möglich ist. Weiterhin kann die oder eine weitere Sensoreinrichtung z. B. zur Erfassung des Gewichts des Köders 2 eingerichtet sein, sodass eine Erfassung eines physikalischen Köderparameters möglich ist. Überdies kann die oder eine weitere Sensoreinrichtung z. B. zur Erfassung der Form bzw. des Volumens des Köders 2 eingerichtet sein, sodass eine Erfassung eines geometrischen Köderparameters möglich ist.

Ein Erfassungselement kann auch eine Leseeinrichtung zur Auslesung einer einen Köderparameter enthaltenden, z. B. köderseitigen, Speichereinrichtung (nicht gezeigt) sein. Die Leseeinrichtung ist datenmäßig mit einer einen Köderparameter enthaltenden, z. B. köderseitigen, Speichereinrichtung verbindbar bzw. verbunden, sodass ein Auslesen des gespeicherten Köderparameters möglich ist. Es ist sonach nicht zwingend notwendig, einen Köderparameter vorrichtungsseitig sensorisch zu erfassen, vielmehr kann ein Köderparameter von der Erfassungseinrichtung 9 ausgelesen und derart erfasst werden.

Ein Erfassungselement kann auch eine Empfangseinrichtung zum Empfang eines, insbesondere von einem Benutzer (vor, während oder nach der Einbringung eines Köders 2 in den Aufnahmeraum 6), übertragenen Köderparameters sein. Die Empfangseinrichtung, welche einen Teil der Ausgabeeinrichtung 11 bilden kann, ist sonach datenmäßig mit einer, insbesondere benutzerseitigen, Sendeeinrichtung, welche einen Teil eines benutzerseitigen (mobilen) Bedien- bzw. Endgeräts bilden kann, verbindbar bzw. bzw. verbunden, sodass ein Empfang eines Köderparameters möglich ist. Es ist sonach auch hier nicht zwingend notwendig, einen Köderparameter vorrichtungsseitig sensorisch zu erfassen, vielmehr kann ein Köderparameter an die Erfassungseinrichtung 9 übertragen werden bzw. von dieser empfangen und derart erfasst werden.

Ein zur Erfassung einer Schädlingsinformation eingerichtetes Erfassungselement kann eine Sensoreinrichtung zur Erfassung von Schädlingen 14 und/oder zur Erfassung von Bewegungen von Schädlingen 14 außerhalb und/oder innerhalb des Aufnahmeraums 6 sein. Die Sensoreinrichtung kann z. B. zur Erfassung einer Atmosphäre und/oder einer Temperatur außerhalb und/oder innerhalb des Aufnahmeraums 6 eingerichtet sein, sodass eine Erfassung eines Schädlingsparameters möglich ist. Beispielsweise kann eine Erfassung einer Atmosphäre mit einer gegenüber einer Referenzatmosphäre veränderten Zusammensetzung (beispielsweise einem erhöhten CO₂-Gehalt) bzw. eine Erfassung einer gegenüber einer Referenztemperatur veränderten Temperatur das Vorhandensein von Schädlingen 14 anzeigen. Die Sensoreinrichtung umfasst hierfür geeignete Gas- bzw. Temperatursensoren. Die Sensoreinrichtung kann jedoch auch zur Erfassung von auf eine Fläche, d. h. z. B. eine Fläche einer Wandung des Gehäuseteils 3 bzw. eine Fläche der Plattformeinrichtung 7, wirkenden Kräften, insbesondere Druck- bzw. Zugkräften, eingerichtet sein, sodass derart eine Erfassung eines Schädlingsparameters möglich ist. Beispielsweise kann eine Erfassung eines auf eine bestimmte Fläche wirkenden Drucks oder Zugs das Vorhandensein von Schädlingen 14 anzeigen. Die Sensoreinrichtung umfasst hierfür geeignete Druck- bzw. Zugsensoren.

In den Fig. ist eine Aufnahmeeinrichtung 15, insbesondere eine Bild- und/oder Tonaufnahmeeinrichtung, zur Aufnahme von Schädlingen 14 und/oder zur Aufnahme von Bewegungen von Schädlingen 14 außerhalb und/oder innerhalb des Aufnahmeraums 6 als konkrete Ausführungsform eines zur Erfassung einer Schädlingsinformation eingerichteten Erfassungselements dargestellt. Die Aufnahmeeinrichtung 15 kann z. B. zur Aufnahme von akustischen und/oder optischen Informationen innerhalb des Aufnahmeraums 6 eingerichtet sein, sodass eine Erfassung eines Schädlingsparameters möglich ist. Beispielsweise kann eine Aufnahme eines bestimmten Geräuschpegels und/oder Bewegungsmusters das Vorhandensein von Schädlingen 14 qualitativ oder quantitativ anzeigen. Die Aufnahmeeinrichtung 15 umfasst hierfür geeignete akustische und/oder optische Aufnahmeelemente, d. h. z. B. Mikrophone und Kameras.

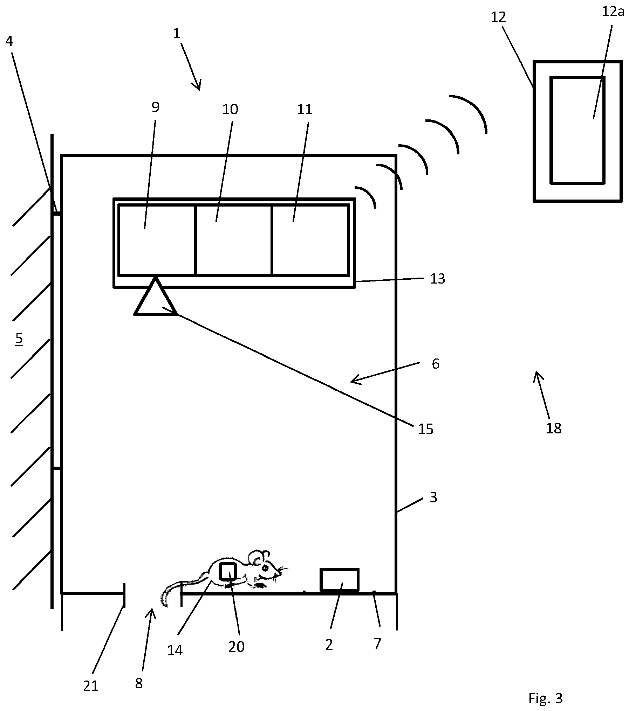

Anhand des in Fig. 3 gezeigten Ausführungsbeispiels ist ersichtlich, dass ein zur Erfassung einer Schädlingsinformation eingerichtetes Erfassungselement auch eine Sensoreinrichtung zur Erfassung einer schädlingsseitigen, d. h. äußerlich oder innerlich mit einem Schädling verbundenen, insbesondere biologischen, chemischen, magnetischen oder mechanischen, Markereinrichtung 20 (Marker) sein kann. Die Sensoreinrichtung kann sonach bestimmte schädlingsseitige Markereinrichtungen 20, welche Kenntnisse über das Vorhandensein bzw. Bewegungsmuster der mit diesen verbundenen Schädlinge 14 liefern, erfassen. Biologische bzw. chemische Markereinrichtungen 20 können bestimmte biologische oder chemische Substanzen sein, welche einem Schädling 14 äußerlich oder innerlich zugeführt worden sind. Beispielsweise kann die Oberfläche eines Schädlings 14 mit einer entsprechenden Substanz benetzt sein. Magnetische oder mechanische Markereinrichtungen 20 können z. B. magnetisch oder mechanisch, d. h. durch einen mechanischen Kontakt, erfassbare magnetische oder mechanische Elemente sein, welche einem Schädling 14 äußerlich oder innerlich zugeführt worden sind. Beispielsweise kann ein entsprechendes Element an einem Körperteil eines Schädlings 14 befestigt sein.

Zur Markierung eins Schädlings 14 mit einer Markereinrichtung 20 kann die Vorrichtung 1 eine Markierungseinrichtung 21 umfassen. Die Markierungseinrichtung 21 ist zur Markierung eines Schädlings 14 mit einer, z. B. biologischen, chemischen, magnetischen oder mechanischen, von der der Erfassungseinrichtung 9 zugehörigen Sensoreinrichtung zur Erfassung einer schädlingsseitigen Markereinrichtung 20 erfassbaren Markereinrichtung 20 eingerichtet. Die Markereinrichtung 20 kann - je nach deren strukturellen Gegebenheiten - z. B. durch Aufsprühen, Berühren eines mit einer Markereinrichtung 20 versehenen, z. B. bürstenartigen, Kontaktbereichs, Spritzen, Anbinden, Ankleben, Anheften, etc. erfolgen. In dem in Fig. 3 gezeigten Ausführungsbeispiel ist eine Markierungseinrichtung 21 im Bereich der Durchgangsöffnung 8 der Plattformeinrichtung 7 angeordnet oder ausgebildet. Die Markierungseinrichtung 21 bildet sonach einen Teil des Gehäuseteils 3.

Prinzipiell kann auch eine außerhalb des Gehäuseteils 3 bzw. der Vorrichtung 1 angeordnete oder ausgebildete Markierungseinrichtung 21 vorgesehen sein.

Ein zur Erfassung einer Schädlingsinformation eingerichtetes Erfassungselement kann auch eine Leseeinrichtung zur Auslesung einer einen Schädlingsparameter enthaltenden, insbesondere schädlingsseitigen, Speichereinrichtung (nicht gezeigt) (Speicherchip, Tag, etc.) sein. Die Leseeinrichtung ist datenmäßig mit dem Schädling 14 bzw. einer einen Schädlingsparameter enthaltenden Speichereinrichtung verbindbar bzw. verbunden, sodass ein Auslesen des Schädlingsparameters möglich ist. Ein Auslesen ist z. B. möglich, wenn sich ein Schädlings 14 innerhalb eines Lesebereichs befindet. Ein solcher Lesebereich kann sich außerhalb oder innerhalb des Gehäuseteils 3 befinden.

Die Vorrichtung 1 kann ferner eine Speichereinrichtung (nicht gezeigt) umfassen. Die Speichereinrichtung ist zur Speicherung vermittels der Erfassungseinrichtung 9 erfasster erster Informationen, d. h. insbesondere Köder- bzw. Schädlingsinformationen, und/oder zur Speicherung vermittels der Steuerungseinrichtung 10 erzeugter zweiter Informationen, d. h. insbesondere Benutzerinformationen, insbesondere Ködergabe- und Schädlingsresistenzinformationen, eingerichtet.

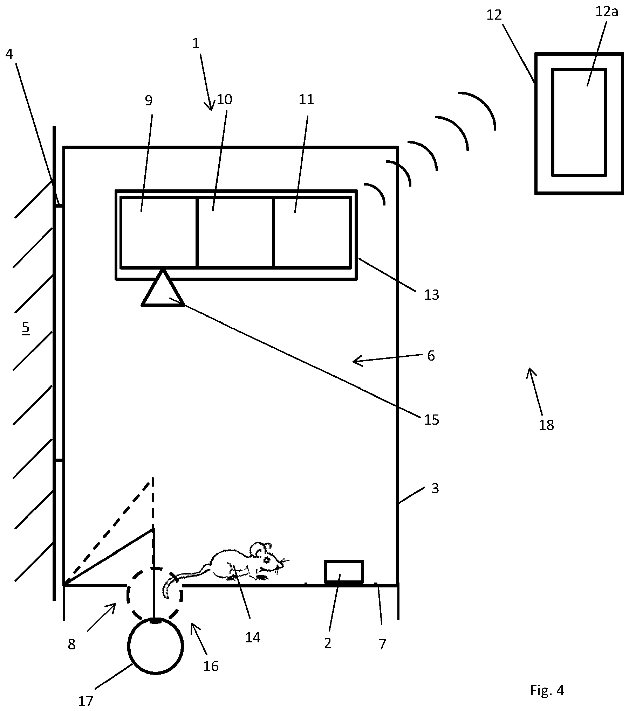

Anhand des in Fig. 4 gezeigten Ausführungsbeispiels ist ersichtlich, dass die Vorrichtung 1 eine Schließeinrichtung 16 umfassen kann. Die Schließeinrichtung 16 umfasst einen an dem Gehäuseteil 3 relativ zu diesem zwischen einer Offenstellung und einer Schließstellung bewegbar gelagerten Schließkörper 17. Der Schließkörper 17 ist in der in Fig. 3 mit durchgezogenem Strich dargestellten Offenstellung derart von einem Zugangsbereich des Gehäuseteils 3 in den Aufnahmeraum 6, d. h. der Durchgangsöffnung 8, weg bewegt, dass der Zugangsbereich geöffnet ist. In der in Fig. 3 mit strichliertem Strich dargestellten Schließstellung ist der Schließkörper 17 derart gegen den Zugangsbereich bewegt, dass der Zugangsbereich, insbesondere hermetisch, verschlossen ist. In der Schließstellung ist eine Kontamination mit den in dem Köder 2 enthaltenen Substanzen ausgeschlossen. Bei dem Schließkörper 17 kann es sich um einen Schwimmkörper handeln, welcher sich bei Anstieg des Wasserspiegels von alleine in die Schließstellung bewegt.

Mit der in den Fig. gezeigten Vorrichtung 1 lässt sich ein Übertragungssystem 18 zur Übertragung von Informationen, insbesondere Benutzerinformationen, bevorzugt Ködergabeinformationen und/oder Schädlingsresistenzinformationen, an wenigstens einen Kommunikationspartner 12 realisieren. Das Übertragungssystem 18 umfasst wenigstens eine Vorrichtung 1 sowie wenigstens einen Kommunikationspartner 12, welcher wenigstens zum Empfang von von der vorrichtungsseitigen Ausgabeeinrichtung 11 übertragener zweiter Informationen eingerichtet ist. Bei dem Kommunikationspartner 12 kann es sich um ein benutzerseitiges (mobiles) End- bzw. Bediengerät, d. h. insbesondere um ein Laptop, ein Smartphone, ein Tablet-PC oder ein sonstiges mobiles elektronisches Gerät handeln.

Mit dem Übertragungssystem 18 lässt sich ein Verfahren zur Übertragung von Informationen, insbesondere Benutzerinformationen, bevorzugt Ködergabeinformationen und/oder Schädlingsresistenzinformationen, an wenigstens einen Übertragungs- bzw. Kommunikationspartner 12 implementieren.

Das Verfahren umfasst in einer ersten Ausgestaltung die folgenden Schritte:
- Erfassen einer ersten Information, insbesondere einer wenigstens einen Köderparameter eines Köders 2 beschreibenden Köderinformation und/oder einer wenigstens einen Schädlingsparameter eines Schädlings 14 beschreibenden Schädlingsinformation, vermittels der Erfassungseinrichtung 9 der Vorrichtung 1 und
- Übertragen der erfassten ersten Information an wenigstens einen Übertragungspartner 12 vermittels der Ausgabeeinrichtung 11 der Vorrichtung 1.

Die datenmäßige Verarbeitung der ersten Information kann hier außerhalb der Vorrichtung 1 erfolgen.

Das Verfahren umfasst einer zweiten Ausgestaltung die folgenden Schritte:
- Erfassen einer ersten Information, insbesondere einer wenigstens einen Köderparameter eines Köders 2 beschreibenden Köderinformation und/oder einer wenigstens einen Schädlingsparameter eines Schädlings 14 beschreibenden Schädlingsinformation, vermittels der Erfassungseinrichtung 9 der Vorrichtung 1,
- Erzeugen einer zweiten Information, insbesondere einer Benutzerinformation, auf Grundlage einer erfassten ersten Information, insbesondere einer Köderinformation und/oder einer erfassten Schädlingsinformation, vermittels der Steuerungseinrichtung 10 der Vorrichtung 1 und
- Übertragen der erzeugten zweiten Information an wenigstens einen Übertragungspartner 12 vermittels der Ausgabeeinrichtung 11 der Vorrichtung 1.

Die datenmäßige Verarbeitung der ersten Informationen kann hier (auch) innerhalb der Vorrichtung 1 erfolgen.

Bestimmte Merkmale der Erfindung sind in den nachfolgenden Aspekten nochmals beispielhaft dargestellt:
1. Vorrichtung (1) zur Halterung eines Köders (2), insbesondere eines Köders (2) für Schädlinge (14), bevorzugt für Nagetiere, umfassend:
   - ein, insbesondere in einen Teil einer Kanalisationsanlage einsetzbares, Gehäuseteil (3) mit einem Aufnahmeraum (6) zur Aufnahme eines Köders (2),
   - eine Erfassungseinrichtung (9), welche zur Erfassung einer ersten Information eingerichtet ist,
   - eine Ausgabeeinrichtung (11), welche zur Ausgabe einer vermittels der Erfassungseinrichtung (9) erfassten ersten Information eingerichtet ist.
2. Vorrichtung nach Aspekt 1, gekennzeichnet durch eine Steuerungseinrichtung (10), welche auf Grundlage einer vermittels der Erfassungseinrichtung (9) erfassten ersten Information zur Erzeugung einer zweiten Information eingerichtet ist, wobei die Ausgabeeinrichtung (11) zur Ausgabe einer vermittels der Steuerungseinrichtung (10) erzeugten zweiten Information eingerichtet ist.
3. Vorrichtung nach Aspekt 1 oder 2, dadurch gekennzeichnet, dass die erste Information eine wenigstens einen Köderparameter eines Köders (2) beschreibende Köderinformation und/oder eine wenigstens einen Schädlingsparameter eines Schädlings (14) beschreibende Schädlingsinformation ist, und
   die zweite Information eine auf Grundlage einer erfassten Köderinformation und/oder einer erfassten Schädlingsinformation erzeugte Benutzerinformation ist.
4. Vorrichtung nach Aspekt 3, dadurch gekennzeichnet, dass die Benutzerinformation eine Ködergabeinformation und/oder eine Köderprüfinformation und/oder eine Schädlingsresistenzinformation ist.
5. Vorrichtung nach Aspekt 4, dadurch gekennzeichnet, dass die Ködergabeinformation eine, insbesondere im Hinblick auf eine bestimmte Zielgröße, insbesondere die Verhinderung der Entwicklung von Resistenzen eines Schädlings (14) gegenüber einem bestimmten Köder (2), zweckmäßige, an einen Benutzer gerichtete qualitative und/oder quantitative Anweisung zur Einbringung eines Köders (2) in den gehäuseteilseitigen Aufnahmeraum (6) beschreibt.
6. Vorrichtung nach Aspekt 4, dadurch gekennzeichnet, dass die Köderprüfinformation eine, insbesondere an eine bestimmte Zeitdauer oder einen bestimmten Zeitpunkt geknüpfte, an einen Benutzer gerichtete qualitative und/oder quantitative Anweisung zur Überprüfung eines gegebenen Köders (2), insbesondere im Hinblick auf durch einen Köderanbiss durch einen Schädling (14) zurückzuführende Veränderungen des Köders (2), beschreibt.
7. Vorrichtung nach Aspekt 4, dadurch gekennzeichnet, dass die Schädlingsresistenzinformation ein aktuelles und/oder künftiges Potential der Entwicklung von Resistenzen eines Schädlings (14) gegenüber einem bestimmten, gegebenenfalls bereits in der Vergangenheit ein- oder mehrfach gegebenen, Köder (2) beschreibt.
8. Vorrichtung nach einem der Aspekte 3 bis 7, dadurch gekennzeichnet, dass der Köderparameter wenigstens einen chemischen und/oder physikalischen und/oder geometrischen Parameter eines in dem gehäuseteilseitigen Aufnahmeraum (6) aufgenommenen Köders (2) qualitativ oder quantitativ beschreibt und/oder wenigstens einen aus einem chemischen und/oder physikalischen und/oder geometrischen Parameter eines in dem gehäuseteilseitigen Aufnahmeraum (6) aufgenommenen Köders (2) abgeleiteten Parameter qualitativ oder quantitativ beschreibt.
9. Vorrichtung nach einem der Aspekte 3 bis 8, dadurch gekennzeichnet, dass der Schädlingsparameter erfasste Schädlinge (14) und/oder Bewegungen eines oder mehrerer erfasster Schädlinge (14) außerhalb und/oder innerhalb des gehäuseteilseitigen Aufnahmeraums (6) qualitativ oder quantitativ beschreibt und/oder wenigstens einen aus erfassten Schädlingen (14) und/oder aus Bewegungen eines oder mehrerer erfasster Schädlinge (14) außerhalb und/oder innerhalb des gehäuseteilseitigen Aufnahmeraums (6) abgeleiteten Parameter qualitativ oder quantitativ beschreibt.
10. Vorrichtung nach einem der Aspekte 3 bis 9, dadurch gekennzeichnet, dass die Erfassungseinrichtung (9) wenigstens ein Erfassungselement zur Erfassung des Köderparameters und/oder des Schädlingsparameters umfasst.
11. Vorrichtung nach Aspekt 10, dadurch gekennzeichnet, dass ein Erfassungselement zur Erfassung eines Köderparameters
   als eine Sensoreinrichtung zur Erfassung eines chemischen und/oder physikalischen und/oder geometrischen Köderparameters, oder
   als eine Leseeinrichtung zur Auslesung einer einen Köderparameter enthaltenden, insbesondere köderseitigen, Speichereinrichtung, oder
   als eine Empfangseinrichtung zum Empfang eines, insbesondere von einem Benutzer, übertragenen Köderparameters ausgebildet ist.
12. Vorrichtung nach einem der Aspekte 3 bis 11, dadurch gekennzeichnet, dass ein Erfassungselement zur Erfassung eines Schädlingsparameters als eine Sensoreinrichtung zur Erfassung von Schädlingen (14) und/oder von Bewegungen eines oder mehrerer Schädlinge (14) außerhalb und/oder innerhalb des gehäuseteilseitigen Aufnahmeraums (6), oder
   als eine Sensoreinrichtung zur Erfassung einer schädlingsseitigen, insbesondere biologischen, chemischen, magnetischen oder mechanischen, Markereinrichtung oder als eine Leseeinrichtung zur Auslesung einer einen Schädlingsparameter enthaltenden, insbesondere schädlingsseitigen, Speichereinrichtung oder
   als eine, insbesondere akustische und/oder optische, Aufnahmeeinrichtung (15) zur Aufnahme von Schädlingen (14) und/oder von Bewegungen eines oder mehrerer erfasster Schädlinge (14) außerhalb und/oder innerhalb des gehäuseteilseitigen Aufnahmeraums (6) ausgebildet ist.
13. Vorrichtung nach einem der vorhergehenden Aspekte, gekennzeichnet durch eine Markierungseinrichtung (19), welche zur Markierung eines Schädlings (14) mit einer, insbesondere biologischen, chemischen, magnetischen oder mechanischen, von der Sensoreinrichtung zur Erfassung einer schädlingsseitigen Markereinrichtung (20) erfassbaren Markereinrichtung (20) eingerichtet ist.
14. Vorrichtung nach einem der vorhergehenden Aspekte, gekennzeichnet durch eine Speichereinrichtung, welche zur Speicherung vermittels der Erfassungseinrichtung (9) erfasster erster Informationen und/oder vermittels der Steuerungseinrichtung (10) erzeugter zweiter Informationen eingerichtet ist.
15. Vorrichtung nach einem der vorhergehenden Aspekte, dadurch gekennzeichnet, dass die Ausgabeeinrichtung (11) als Signalausgabeeinrichtung zur direkten Ausgabe von erzeugte zweite Informationen beschreibenden Signalen an wenigstens einen Benutzer ausgebildet ist, oder
   die Ausgabeeinrichtung (11) als Übertragungseinrichtung ausgebildet ist, welche zur, insbesondere funkbasierten, Übertragung vermittels der Erfassungseinrichtung (9) erfasster erster Informationen und/oder vermittels der Steuerungseinrichtung (10) erzeugter zweiter Informationen an wenigstens einen Übertragungspartner (12), insbesondere an ein benutzerseitiges Bedien- oder Endgerät und/oder an ein lokales oder globales Datennetzwerk, eingerichtet ist.
16. Vorrichtung nach einem der vorhergehenden Aspekte, dadurch gekennzeichnet, dass die Erfassungseinrichtung (9) und/oder die Steuerungseinrichtung (10) und/oder die Ausgabeeinrichtung (11) und/oder die Markierungseinrichtung (19) einen integrierten Bestandteil des Gehäuseteils (3) bildet, oder
   die Erfassungseinrichtung (9) und/oder die Steuerungseinrichtung (10) und/oder die Ausgabeeinrichtung (11) und/oder die Markierungseinrichtung (19) einen integrierten Bestandteil eines, gegebenenfalls mit dem Gehäuseteil (3) verbindbaren oder verbundenen oder zu dem Gehäuseteil (3) gesonderten, Bauteils (13) bildet.
17. Vorrichtung nach einem der vorhergehenden Aspekte, gekennzeichnet durch eine Schließeinrichtung (16), welche einen, insbesondere an dem Gehäuseteil (3), relativ zu dem Gehäuseteil (3) zwischen einer Offenstellung und einer Schließstellung bewegbar gelagerten Schließkörper (17) umfasst, wobei der Schließkörper (17) in der Offenstellung derart von einem Zugangsbereich in den gehäuseteilseitigen Aufnahmeraum (6) weg bewegt ist, dass der Zugangsbereich geöffnet ist, und in einer Schließstellung derart gegen den Zugangsbereich bewegt ist, dass der Zugangsbereich, insbesondere hermetisch, verschlossen ist.
18. Übertragungssystem (18) zur Übertragung von Informationen an wenigstens einen Übertragungspartner (12), umfassend:
   - wenigstens eine Vorrichtung (1) zur Halterung eines Köders (2) nach einem der vorhergehenden Aspekte,
   - wenigstens einen Übertragungspartner (12), welcher wenigstens zum Empfang von von der vorrichtungsseitigen Ausgabeeinrichtung (11) übertragener zweiter Informationen eingerichtet ist.
19. Verfahren zur Übertragung von Informationen an wenigstens einen Übertragungspartner (12), gekennzeichnet durch die folgenden Schritte:
   - Erfassen einer ersten Information, insbesondere einer wenigstens einen Köderparameter eines Köders (2) beschreibenden Köderinformation und/oder einer wenigstens einen Schädlingsparameter eines Schädlings (14) beschreibenden Schädlingsinformation, vermittels einer Erfassungseinrichtung (9) einer Vorrichtung (1) zur Halterung eines Köders (2) nach einem der Aspekte 1-17 und
   - Übertragen der erfassten ersten Information an wenigstens einen Übertragungspartner (12) vermittels einer Ausgabeeinrichtung (11) der Vorrichtung (1) zur Halterung eines Köders nach einem der Aspekte 1 - 17, oder
   - Erfassen einer ersten Information, insbesondere einer wenigstens einen Köderparameter eines Köders (2) beschreibenden Köderinformation und/oder einer wenigstens einen Schädlingsparameter eines Schädlings (14) beschreibenden Schädlingsinformation, vermittels einer Erfassungseinrichtung (9) einer Vorrichtung (1) zur Halterung eines Köders (2) nach einem der Aspekte 1-17,
   - Erzeugen einer zweiten Information, insbesondere einer Benutzerinformation, auf Grundlage einer erfassten ersten Information, insbesondere einer Köderinformation und/oder einer erfassten Schädlingsinformation, vermittels einer Steuerungseinrichtung (10) der Vorrichtung (1) zur Halterung eines Köders (2) nach einem der Aspekte 1-17 und
   - Übertragen der erzeugten zweiten Information an wenigstens einen Übertragungspartner (12) vermittels einer Ausgabeeinrichtung (11) der Vorrichtung (1) zur Halterung eines Köders nach einem der Aspekte 1-17.

## Patentansprüche

1. Vorrichtung (1) zur Halterung eines Köders (2), insbesondere eines Köders (2) für Schädlinge (14), bevorzugt für Nagetiere, umfassend:
- ein, insbesondere in einen Teil einer Kanalisationsanlage einsetzbares, Gehäuseteil (3) mit einem Aufnahmeraum (6) zur Aufnahme eines Köders (2),
- eine Erfassungseinrichtung (9), welche zur Erfassung einer ersten Information eingerichtet ist,
- eine Ausgabeeinrichtung (11), welche zur Ausgabe einer vermittels der Erfassungseinrichtung (9) erfassten ersten Information eingerichtet ist.

2. Vorrichtung nach Anspruch 1, **gekennzeichnet durch** eine Steuerungseinrichtung (10), welche auf Grundlage einer vermittels der Erfassungseinrichtung (9) erfassten ersten Information zur Erzeugung einer zweiten Information eingerichtet ist, wobei die Ausgabeeinrichtung (11) zur Ausgabe einer vermittels der Steuerungseinrichtung (10) erzeugten zweiten Information eingerichtet ist.

3. Vorrichtung nach Anspruch 1 oder 2, **dadurch gekennzeichnet, dass** die erste Information eine wenigstens einen Köderparameter eines Köders (2) beschreibende Köderinformation und/oder eine wenigstens einen Schädlingsparameter eines Schädlings (14) beschreibende Schädlingsinformation ist, und
die zweite Information eine auf Grundlage einer erfassten Köderinformation und/oder einer erfassten Schädlingsinformation erzeugte Benutzerinformation ist.

4. Vorrichtung nach Anspruch 3, **dadurch gekennzeichnet, dass** die Benutzerinformation eine Ködergabeinformation und/oder eine Köderprüfinformation und/oder eine Schädlingsresistenzinformation ist.

5. Vorrichtung nach Anspruch 4, **dadurch gekennzeichnet, dass** die Ködergabeinformation eine, insbesondere im Hinblick auf eine bestimmte Zielgröße, insbesondere die Verhinderung der Entwicklung von Resistenzen eines Schädlings (14) gegenüber einem bestimmten Köder (2), zweckmäßige, an einen Benutzer gerichtete qualitative und/oder quantitative Anweisung zur Einbringung eines Köders (2) in den gehäuseteilseitigen Aufnahmeraum (6) beschreibt.

6. Vorrichtung nach Anspruch 4, **dadurch gekennzeichnet, dass** die Köderprüfinformation eine, insbesondere an eine bestimmte Zeitdauer oder einen bestimmten Zeitpunkt geknüpfte, an einen Benutzer gerichtete qualitative und/oder quantitative Anweisung zur Überprüfung eines gegebenen Köders (2), insbesondere im Hinblick auf durch einen Köderanbiss durch einen Schädling (14) zurückzuführende Veränderungen des Köders (2), beschreibt.

7. Vorrichtung nach Anspruch 4, **dadurch gekennzeichnet, dass** die Schädlingsresistenzinformation ein aktuelles und/oder künftiges Potential der Entwicklung von Resistenzen eines Schädlings (14) gegenüber einem bestimmten, gegebenenfalls bereits in der Vergangenheit ein- oder mehrfach gegebenen, Köder (2) beschreibt.

8. Vorrichtung nach einem der Ansprüche 3 bis 7, **dadurch gekennzeichnet, dass** der Köderparameter wenigstens einen chemischen und/oder physikalischen und/oder geometrischen Parameter eines in dem gehäuseteilseitigen Aufnahmeraum (6) aufgenommenen Köders (2) qualitativ oder quantitativ beschreibt und/oder wenigstens einen aus einem chemischen und/oder physikalischen und/oder geometrischen Parameter eines in dem gehäuseteilseitigen Aufnahmeraum (6) aufgenommenen Köders (2) abgeleiteten Parameter qualitativ oder quantitativ beschreibt.

9. Vorrichtung nach einem der Ansprüche 3 bis 8, **dadurch gekennzeichnet, dass** der Schädlingsparameter erfasste Schädlinge (14) und/oder Bewegungen eines oder mehrerer erfasster Schädlinge (14) außerhalb und/oder innerhalb des gehäuseteilseitigen Aufnahmeraums (6) qualitativ oder quantitativ beschreibt und/oder wenigstens einen aus erfassten Schädlingen (14) und/oder aus Bewegungen eines oder mehrerer erfasster Schädlinge (14) außerhalb und/oder innerhalb des gehäuseteilseitigen Aufnahmeraums (6) abgeleiteten Parameter qualitativ oder quantitativ beschreibt.

10. Vorrichtung nach einem der Ansprüche 3 bis 9, **dadurch gekennzeichnet, dass** die Erfassungseinrichtung (9) wenigstens ein Erfassungselement zur Erfassung des Köderparameters und/oder des Schädlingsparameters umfasst.

11. Vorrichtung nach einem der vorhergehenden Ansprüche, **gekennzeichnet durch** eine Markierungseinrichtung (21), welche zur Markierung eines Schädlings (14) mit einer, insbesondere biologischen, chemischen, magnetischen oder mechanischen, von der Sensoreinrichtung zur Erfassung einer schädlingsseitigen Markereinrichtung (20) erfassbaren Markereinrichtung (20) eingerichtet ist.

12. Vorrichtung nach Anspruch 11, **dadurch gekennzeichnet, dass** die Markierungseinrichtung (21) eingerichtet ist, eine Markierung durch Aufsprühen, Berühren eines mit einer Markereinrichtung (20) versehenen, z. B. bürstenartigen, Kontaktbereichs, Spritzen, Anbinden, Ankleben, Anheften, etc., aufzubringen.

13. Vorrichtung nach Anspruch 11 oder 12, **dadurch gekennzeichnet, dass** die Markierungseinrichtung (21) einen Teil des Gehäuseteils (3) bildet oder außerhalb des Gehäuseteils (3) angeordnet ist.

14. Vorrichtung nach einem der vorhergehenden Ansprüche, **dadurch gekennzeichnet, dass** die Ausgabeeinrichtung (11) als Signalausgabeeinrichtung zur direkten Ausgabe von erzeugte zweite Informationen beschreibenden Signalen an wenigstens einen Benutzer ausgebildet ist, oder
die Ausgabeeinrichtung (11) als Übertragungseinrichtung ausgebildet ist, welche zur, insbesondere funkbasierten, Übertragung vermittels der Erfassungseinrichtung (9) erfasster erster Informationen und/oder vermittels der Steuerungseinrichtung (10) erzeugter zweiter Informationen an wenigstens einen Übertragungspartner (12), insbesondere an ein benutzerseitiges Bedien- oder Endgerät und/oder an ein lokales oder globales Datennetzwerk, eingerichtet ist.

15. Vorrichtung nach einem der vorhergehenden Ansprüche, **dadurch gekennzeichnet, dass** die Erfassungseinrichtung (9) und/oder die Steuerungseinrichtung (10) und/oder die Ausgabeeinrichtung (11) und/oder die Markierungseinrichtung (21) einen integrierten Bestandteil des Gehäuseteils (3) bildet, oder
die Erfassungseinrichtung (9) und/oder die Steuerungseinrichtung (10) und/oder die Ausgabeeinrichtung (11) und/oder die Markierungseinrichtung (21) einen integrierten Bestandteil eines, gegebenenfalls mit dem Gehäuseteil (3) verbindbaren oder verbundenen oder zu dem Gehäuseteil (3) gesonderten, Bauteils (13) bildet.
